# EUROPÄISCHE PATENTSCHRIFT

(11) **EP 1 258 526 B1**
(45) Veröffentlichungstag und Bekanntmachung des Hinweises auf die Patenterteilung: **02.08.2006**
(21) Anmeldenummer: 01111777.7
(22) Anmeldetag: 15.05.2001
(51) Int. Cl.: C12N 1/18, C12R 1/225, A21D 8/04

(54) **Verfahren zur Herstellung eines Sauerteiges unter Verwendung von Hefe und homo- und heterofermentativen Laktobazillen**
Process for preparing a sourdough with yeast and homo- and heterofermentative Lactobacilli
Procédé pour la préparation d'un levain de panification à partir de levure et de Lactobacilles homo- et hétérofermentaires

(43) Veröffentlichungstag der Anmeldung: 20.11.2002
(73) Patentinhaber: Ernst Böcker GmbH & Co. KG, 32427 Minden (DE)
(72) Erfinder: Stolz, Peter, Dr., 32457 Porta Westfalica (DE)
(74) Vertreter: Thiel, Christian

(56) Entgegenhaltungen:
- EP-A- 0 636 692
- EP-A- 0 953 288
- US-A- 5 108 766
- US-A- 5 700 684
- PATENT ABSTRACTS OF JAPAN vol. 2000, no. 10, 17. November 2000 (2000-11-17) & JP 2000 189041 A (TAKAKI BEEKARII:KK), 11. Juli 2000 (2000-07-11)

## Beschreibung

Die Erfindung betrifft ein Verfahren zur Herstellung eines Starters zur Weizenvorteig- und Sauerteigherstellung unter Verwendung von Wasser und mindestens eines Weizenmahlproduktes, welches durch Zusatz eines Laktobazillen (Milchsäurebakterien) und Hefen enthaltenden Impfgutes teilweise vergoren wird.

Die Erfindung betrifft ferner Mikroorganismen sowie Mischkulturen, welche Laktobazillen und Hefen enthalten, die sich für die Durchführung des Verfahrens in besonderem Maße eignen.

Die Erfindung betrifft darüber hinaus ein Verfahren zur Herstellung von Weizenvorteigen und Weizensauerteigen sowie daraus hergestellten Produkten zur direkten Herstellung von Backwaren.

Während bei der Herstellung von auf Roggen basierenden Brotteigen die Verwendung von Sauerteig in erster Linie der Säuerung und der Lockerung des Teiges dient, steht bei der Verwendung von Weizenvor- und Weizensauerteigen für die Weizenbrot- und Weizenmischbrotherstellung die Aroma- und Geschmacksgebung im Vordergrund.

Traditionell werden Weizenvorteigführungen überwiegend mit Backhefen beimpft, welche häufig mit Milchsäurebakterien verunreinigt sind. Diese vermeintlichen Kontaminationen führen bei Vorteigführungen mit längerer Stehzeit zu einer Säurebildung, die sich insgesamt positiv auf die Geschmacksgebung auswirken kann. Die in Backhefen gefundenen Milchsäurebakterien gehören jedoch meist zu Milchsäurebakterien-Spezies, die an das Habitat "Teig" nicht oder nur unzureichend angepaßt sind, wodurch eine reproduzierbare Vorteigqualität nicht gewährleistet werden kann.

Der Begriff Starter bezieht sich im folgenden auf das gesamte Verfahrensprodukt, welches unter anderem die noch nicht, teil- oder vollvergorenen Substrate (das oder die Getreidemahlprodukte) sowie die generierte mikrobielle Flora und die von dieser erzeugten Aromapräkursoren, etc. enthält. Unter dem Begriff "Starterkultur" wird dagegen die generierte mikrobielle Mischkultur selbst verstanden.

Der Begriff "Impfgut" kann sich dabei sowohl auf Starter als auch auf Starterkulturen beziehen.

Als Weizenvorteige werden im folgenden Teige bezeichnet, welche nach Inokulation mit Mikroorganismen bereits teilvergoren sind, jedoch einen höheren pH-Wert (pH ≥ 4,5) und niedrigere Säuregrade (Sr°) (Sr° < 12) aufweisen als Sauerteige. Als Weizensauerteige werden im folgenden länger geführte Weizenvorteige mit höheren Säuregraden als Weizenvorteige (Sr° > 12) und niedrigeren pH-Werten (pH < 4,5) und anderer Zusammensetzung der mikrobiellen Flora verstanden.

Sauerteig- bzw. Vorteigfermentationen sind nicht auf die bloße Säuerung und Gasbildung im Teig beschränkt. Auch Quellungsvorgänge und vor allem die Bildung von Aromapräkursoren aus dem Eiweiß-, Fett- und Kohlenhydratstoffwechsel der beteiligten Milchsäurebakterien oder Milchsäurebakterien und Hefen tragen wesentlich zur Qualität von Backwaren bei. Die in den Vorstufen (Vorteig oder Sauerteig) gebildeten Aromapräkursoren werden später im Backprozeß in Aromastoffe umgewandelt, welche den vorteig- bzw. sauerteigtypischen Geschmack des Gebäckes wesentlich bestimmen.

Ein weiterer positiver Effekt der Verwendung von gesäuerten Vorstufen (Vorteig oder Sauerteig) ist die Unterdrückung des Wachstums von Schimmelpilzen und fadenziehenden Bakterien, wodurch die Haltbarkeit und Lagerstabilität von Gebäcken im Vergleich zu ungesäuerten bzw. nicht ausreichend gesäuerten Gebäcken deutlich verbessert ist.

Da die Herstellung von hochwertigen fermentierten Vorstufen (Vorteige und Sauerteige) zeit- und arbeitsaufwendig ist und darüberhinaus erfahrenes und geschultes Personal bedarf, werden zur Sicherstellung der Starter-Qualität der Anstellgüter in Bäckerein häufig kommerziell erworbene Sauerteigstarter bzw. Anstellgüter eingesetzt, um eine gleichmäßige Sauerteigqualität und dadurch bedingte gleichbleibende Qualität der hergestellten Backwaren in Bezug auf Aroma, Geschmack, Textur und Farbe der Krume und Kruste zu gewährleisten.

Sauerteig- und Vorteig-Fermentationen können spontan oder mit Hilfe von geeigneten Starterkulturen bzw. Anstellgütern eingeleitet werden. Spontan eingeleitete Fermentationsprozesse unterliegen dabei immer einem höheren mikrobiologischen Risiko: So kann es in Folge einer fehlenden schnellen Absenkung des pH-Wertes durch fehlende Aktivität von an das Substrat Teig adaptierten Milchsäurebakterien zu Fehlgärungen durch die im Mehl immer vorhandene Fremdflora (z.B. Enterobakterien, Gelbkeime, Schimmelpilze, Kahmhefen) kommen.

Um eine gleichbleibende Vorteig-/Sauerteig-Qualität zu gewährleisten, ist es im Stand der Technik bekannt, Starterkulturen durch technologische "Reinkulturverfahren" unter sterilen Bedingungen im Fermenter zu gewinnen. Diese Reinkulturen sind zwar einfach und reproduzierbar zu kultivieren aber häufig nicht an die Bedingungen in der bäckerüblichen Praxis (s.u.) angepasst. Dadurch sind diese Reinkulturen unsicher im Durchsetzungsvermögen, wodurch es zu qualitativen Schwankungen in der Qualität von Vor- und Sauerteigen und somit auch der daraus hergestellten Backwaren kommen kann.

Ein weiterer Nachteil der technologischen Reinkulturverfahren besteht darin, daß diese Verfahren den Anforderungen an die Produktion ökologischer Backwaren nicht gewachsen sind: Da sich die Kultivierungs- und Zuchtmedien der Reinkulturen nicht zum Einsatz bei der Brotherstellung eignen, müssen die Mikroorganismen zunächst vom Kulturmedium abgetrennt werden, so daß das überschüssige Medium als Abfall entsorgt werden muß. Dies gilt sowohl für die Herstellung von Milchsäurebaketrien in Form von Rein- oder Misch-Kulturen wie auch für die Herstellung sogenannter Biohefe gemäß dem in der Schrift US 5 700 684 beschriebenen Verfahren (s.u.).

Bäckerübliche Führungsweisen bei Sauerteigen sind im wesentlichen dadurch charakterisiert, daß ein Sauerteig nur einmal monatlich bis einmal wöchentlich mit einer Starterkultur bzw. Anstellgut angeimpft bzw. angefrischt wird. Über den Rest des Monats bzw. der Woche werden die Sauerteige, "Sauerteig auf Sauerteig" geführt, das heißt von einem reifen Sauerteig wird ein Anstellgut als Impfgut für den Sauerteig des nächsten Tages abgenommen. Trotz dieser Verfahrensweise (innerbetriebliche Anstellgut-Herstellung) ist es in der Praxis häufig notwendig, Betriebsauerteige mit einer neuen Sauerteigkultur zu beimpfen, um eine gleichmäßige Sauerteigqualität gewährleisten zu können. Hierzu ist es gängige Praxis auf kommerziell hergestellte Starterkulturen oder Anstellgüter zurückzugreifen.

Für Weizenvor- und Weizensauerteige gab es bisher kein kommerziell erhältliches Produkt, das nach dem Prinzip der "natürlichen Reinzucht im Lebensmittel" hergestellt wurde und somit eine Mikroflora enthält, die den Anforderungen der bäckerüblichen Praxis stand hält und eine reproduzierbare Qualität des Weizenvorteiges bzw. mild gesäuerten Weizensauerteiges garantiert.

Aus der Schrift US 5 700 684 ist ein Verfahren zur Herstellung einer Biomasse aus Laktobazillen und Hefen bekannt, welche ohne die vorherige Abtrennung der Organismen vom Kulturmedium zur Brotherstellung eingesetzt werden. Zu diesem Zweck wird ein Kulturmedium verwendet, welches durch enzymatische Teilumsetzung eines wäßrigen Vollkornmehlgemisches zubereitet wird, ohne daß ein Zusatz chemischer Additive nötig ist. Das Kulturmedium wird mit Hefe und Laktobazillen angeimpft und über mehrere Stunden fermentiert.

Zwar wird hier eine Kokultivierung verschiedener Laktobazillenstämme mit einer Hefe beschrieben, jedoch handelt es sich dabei um die willkürliche Vermengung verschiedener zuvor unter artifiziellen Laborbedingungen in Monokultur gehaltener Reinzuchtstämme, deren Durchsetzungsfähigkeit im Teig unter Bäckereibedingungen fraglich ist. Überdies handelt es sich bei der eingesetzten Hefe nicht um einen an das gesäuerte Milieu des Sauerteiges bzw. Vorteiges angepaßten Stamm, so daß zur Gewährleistung einer guten Triebkraft des Brotteiges die Zugabe von Backhefe notwendig ist.

Angesichts der mit dem Stand der Technik verbundenen Nachteile besteht die Aufgabe der Erfindung darin, die Herstellung hochwertiger Weizenbackwaren auf der Basis von Weizenvorteigen bzw. mild gesäuerten Weizensauerteigen unter weitestgehendem Ausschluß von Qualitätsschwankungen zu ermöglichen, die den Anforderungen an eine wirklich ökologische Lebensmittelherstellung standhält.

Diese Aufgabe wird erfindungsgemäß gelöst durch ein Verfahren zur Herstellung eines Starters für die Weizenvorteig und Weizensauerteigherstellung unter Verwendung von Wasser und mindestens eines Weizenmahlproduktes, welches durch Zusatz eines Laktobazillen und Hefen enthaltenden Impfgutes teilweise vergoren wird, wobei das Impfgut eine adaptierte Mischflora aufweist, welche mindestens einen Hefestamm sowie eine Laktobazillenflora mit mindestens jeweils einem Stamm homo- und heterofermentativer Laktobazillen enthält.

Unter dem Begriff der "adaptierten Mischflora" wird erfindungsgemäß eine an die Kulturbedingungen im vergärenden Getreidemahlprodukt-Wasser Gemisch angepaßte Mikroorganismenflora verstanden, welche sich bei fortwährender Kokultivierung eines Gemisches unterschiedlicher Mikroorganismen bei bestimmten Bedingungen im Habitat Vorteig/Sauerteig herausbildet. Dabei sind auch die einzelnen, in der adaptierten Mikroorganismenflora enthaltenen Hefen bzw. Laktobazillen an die Milieubedingungen im sich bildenden Sauerteig (hinsichtlich pH-Wert, Säureresistenz, Nährstoffquellen, etc.) besonders angepaßt. Es handelt sich dabei um spezielle Sauerteigorganismen ("Sauerteiglaktobazillen" bzw. "Sauerteighefen"). Der auf Basis dieser Mikroorganismen hergestellte Starter weist eine Flora auf, die auch unter unsterilen Bäckereibedingungen über einen möglichst großen Zeitraum stabil ist, während dem der Bäcker den kommerziell erworbenen Starter zur Herstllung von Weizenvor- und Weizensauerteigen nutzt und bäckerüblich, das heißt Vorteig auf Vorteig/Sauerteig oder Sauerteig auf Vorteig/Sauerteig, insbesondere Vorteig auf Vorteig und Sauerteig auf Sauerteig, bis zum Bezug eines neuen Starters weiterführt.

Das erfindungsgemäße Herstellungsverfahren ermöglicht somit die Herstellung qualitativ hochwertiger Starter unter Minimierung qualitativer Schwankungen.

Bei dem erfindungsgemäß hergestellten Starter handelt es sich um ein aktives Anstellgut. Er eignet sich demgemäß besonders zur indirekten Herstellung von Backwaren auf Weizenbasis, d.h. zur Herstellung von Weizenvorteigen und Weizensauerteigen, insbesondere mild gesäuerten Weizensauerteigen, die dann dem Teig der herzustellenden Backware zur Teighebung, -Lockerung und -Aromatisierung, etc. zugesetzt werden (indirekte Herstellungsweise). Überdies können aus den mit Hilfe des erfindungsgemäß hergestellten Starters hergestellten Weizenvor- und Weizensauerteigen Weizenvorteig- und Weizensauerteigprodukte hergestellt werden, in denen der Stoffwechsel und die Vermehrung der Mikroorganismen durch geeignete Maßnahmen unterbunden werden. Solche Weizenvorteig- und Weizensauerteigprodukte werden dann zur direkten Backwarenherstellung eingesetzt. Sie dienen der Aromatisierung, Quellung und besseren Maschinengängigkeit des Teiges. Da Stoffwechsel und Vermehrung der darin enthaltenen Mikroorganismen unterbunden sind und im wesentlichen keine Fermentierung mehr bewirken können, müssen dem Teig der herzustellenden Backware zur Teighebung stoffwechselaktive Mikroorganismen (in der Regel Hefen) zugesetzt werden.

Aufgrund seiner Aktivität und seiner durchsetzungsfähigen Mikroorganismen eignet sich der Starter auch besonders gut zur Herstellung neuer Starter und Starterkulturen.

Der Einsatz eines Impfgutes mit einer adaptierten Mischflora bei der Durchführung des erfindungsgemäßen Verfahrens ermöglicht die reproduzierbare Herstellung von qualitativ hochwertigen Weizenvorteigen und Weizensauerteigen in Bäckereien. Durch den Einsatz einer adaptierten und säureresistenten Hefe wird ein sehr hefereiches Anstellgut für Vorteige bzw.

Sauerteige hervorgebracht, die zudem unter Vorteig bzw. Sauerteig-Bedingungen noch vermehrungsfähig und dadurch besonders triebstark ist.

Die Säuerung bei Sauerteigen und Vorteigen basiert auf dem Kohlenhydrat-Stoffwechsel von Milchsäurebakterien, im wesentlichen Laktobazillen, die aufgrund ihrer Stoffwechseleigenschaften als homo- oder heterofermentative Laktobazillen klassifiziert werden.

Die Gasentwicklung beruht in den meisten Sauerteigen auf der Stoffwechselaktivität von Hefen und heterofermentativen Laktobazillen. In milden Weizensauerteigen beruht die hauptsächliche Gasbildung auf der Stoffwechseltätigkeit geeigneter Sauerteighefen, die eine gewisse Säuretoleranz und Substratvorliebe aufweisen müssen. Die im Stand der Technik bei der Backwarenherstellung eingesetzten Backhefen sind jedoch im Habitat Vor- bzw. Sauerteig nicht mehr vermehrungsfähig, so daß sie sich in bäckerüblich geführten Vor- und Sauerteigen nicht durchsetzen. Im Gegensatz zu den üblichen Backhefen (Saccharomyces cerevisiae Stämme, die keine ausgeprägte Säuretoleranz aufweisen und Maltose-positiv sind) haben die erfindungsgemäßen Vorteig- oder Sauerteighefekulturen, die durch das Verfahren der natürlichen Reinzucht gewonnen werden, die Eigenschaft, sich auch unter praxisgerechten Bedingungen bäckerüblicher Sauerteig- und Vorteigführungen in den Sauerteigen/Vorteigen zu halten und zu vermehren, d.h. sie können sich unter diesen Bedingungen durchsetzen.

Sauerteig-Mikrofloren setzen sich aus drei unterschiedlichen Gruppen von Mikroorganismen zusammen:
1. Homofermentative Laktobazillen, die Glukose fast ausschließlich zu Milchsäure Verstoffwechseln.
2. Heterofermentative Laktobazillen, die Glukose bzw. Maltose aufgrund einer anderen enzymatischen Ausstattung zu Milchsäure, CO₂ und Ethanol oder Essigsäure verstoffwechseln. Sauerteige, die aus überwiegend heterofermentativen Laktobazillen hergestellt werden, weisen in der Regel niedrigere Säuregrade auf, als Sauerteige, die überwiegend mit homofermentativen Laktobazillen hergestellt wurden. In sensorischer Hinsicht sind jedoch die mit heterofermentativen Laktobazillen hergestellten Sauerteige denen mit homofermentativen Laktobazillen hergestellten überlegen.
3. Ein weiterer sehr wichtiger Bestandteil von Sauerteigen/Vorteigen sind säureresistente Hefen, die wesentlich zur Triebkraft des Brotteiges beitragen und darüber hinaus das Aroma und den Geschmack der Backprodukte positiv beeinflussen.

Gemäß neuester Erkenntnisse in der Sauerteigforschung kann die typische Mikroflora von Sauerteigen in drei Klassen eingeteilt werden (Böcker, G., P., Stolz & W. P., Hammes (1995). Neue Erkenntnisse zum Ökosystem Sauerteig und zur Physiologie der sauerteigtypischen Stämme Lactobacillus sanfrancisco und Lactobacillus pontis. Getreide, Mehl und Brot 49/6, 370-374. Stolz, P. (1999). Handbuch Sauerteig. Behr's Verlag Hamburg, 35-60. Mikrobiologie des Sauerteiges. In Spicher G. and Stephan H. (Eds.):

Die Mikroflora von Typ-I-Sauerteigen sind Mikrofloren von Sauerteigen oder pastösen Sauerteigstarter-Präparaten (Anstellgüter) aus bäckerüblich fortlaufend geführten Sauerteigen, deren Bakterienflora in erster Linie von heterofermentativen Laktobazillen der Spezies *Lactobacillus sanfranciscensis* und *Lb. pontis* dominiert werden. In Typ-I-Sauerteigen sind diese sauerteigtypischen Milchsäurebakterien sehr häufig mit säureresistenten Maltose-negativen Hefen wie z.B. *Candida milleri* vergesellschaftet.

Typ-II-Sauerteige sind flüssige Sauerteige (Teigausbeuten > 200), die über einen Zeitraum von mehreren Tagen fermentiert werden. Durch wiederholtes Anfrischen und einer fortlaufenden Führungsweise stellen sich in Abhängigkeit der Temperatur und der verwendeten Rohstoffe selektive Bedingungen ein, die das Wachstum von homofermentativen Stämmen der Spezies *Lb. amylovorus, Lb. crispatus, Lb. farciminis* und heterofermentativen Stämmen der Spezies *Lb. pontis, Lb. reuteri, Lb. panis* und *Lb. fermentum*-ähnlichen Species usw. begünstigen.

Bei der Mikroflora von Typ-III-Sauerteigen handelt es sich Mikroorganismen von getrockneten Sauerteigen oder Sauerteigstarter-Präparaten in getrockneter Form (Pulver), deren Flora in erster Linie aus trocknungsresistenten Organismen wie z.B. *Lb. plantarum, Lb. brevis* sowie *Pediococcus pentosaceus* bestehen.

Aufgrund mangelnder Kenntnisse über die mikrobiologischen Zusammenhänge in bäckerüblich geführten Vorteigen war es bisher nicht möglich, Starterkulturen/Anstellgüter anzubieten, mit denen sich mikrobiologisch stabile Vorteige mit Hilfe bäckerüblicher Führungsweisen (s.o.) herstellen ließen. Der hier vorliegenden Erfindung liegt ein Verfahren zugrunde, das die reproduzierbare Herstellung eines Vorstufen-Starters (Vorteig-/Sauerteig-Starters bzw. Anstellgutes) im Lebensmittel selbst erlaubt. Mit Hilfe dieses Verfahrens der "natürlichen Reinzucht im Lebensmittel" kann eine Mischflora aus homo- und heterofermentativen Laktobazillen und Sauerteighefen gewonnen werden, die eine sehr hohe Aktivität und trotzdem milde Säuerungseigenschaften sowie ein sehr hohes Aromapotential besitzt.

Durch das erfindungsgemäße Verfahren wird erstmals die reproduzierbare Herstellung eines Weizenstarters für Weizenvorteige und Weizensauerteige, insbesondere mild gesäuerte Weizensauerteige möglich, welcher eine an die Praxisbedingungen adaptierte Mischkultur mit an Vorteig- bzw. Sauerteigbedingungen adaptierten Sauerteigmikroorganismen aufweist und sich besonders zur Verwendung als Vorstufen-Starter bzw. Anstellgut für bäckerüblich geführte Weizenvorteige und Weizensauerteige eignet.

Es ist besonders zweckmäßig, wenn als Hefe ein von S. cerevisiae abgeleiteter Sauerteigstamm und besonders bevorzugt der Stamm Saccharomyces species DSM 14265 in der adaptierten Mischflora des Impfgutes enthalten ist. Es handelt sich dabei um eine spezielle Vorteig-/Sauerteighefe, die auch im Vor-/bzw. Sauerteig noch stoffwechselaktiv und vermehrungsfähig ist und sich besonders für die Herstellung von Startern für Weizenvor-/bzw. Weizensauerteige eignet und so im Gegensatz zu herkömmlichen Bäckerhefen besonders zur indirekten Backwarenherstellung geeignet ist, bei der eine Vermehrung der Mikroorganismen im Teig notwendig ist.

Es ist überdies vorteilhaft, wenn die Laktobazillenflora des Impfgutes wenigstens drei der folgenden Laktobazillenstämme enthält: Lb. plantarum DSM 14268, Lb. pontis I DSM 14269, Lb. sanfranciscensis DSM 14270, Lb. crispatus DSM 14271, Lb. pontis II DSM 14272, Lb. pontis III DSM 14273, Lb. pontis IV DSM 14274. Diese Stämme sind ebenso besonders an das Habitat von Weizenvorund Weizensauerteigen, insbesondere mild gesäuerten Weizensauerteigen angepaßt und eignen sich auch aufgrund ihrer Vermehrungsfähigkeit sowie ihrer Produktionsprofile bezüglich Säuren und Aromapräkursoren insbesondere für die Herstellung von Startern für Weizenvor- und Weizensauerteigen zum Einsatz bei der indirekten Backwarenherstellung. Die vorbezeichneten Stämme (Hefe und Laktobazillen) sind bei der DSMZ Braunschweig gemäß Budapester Vertrag hinterlegt.

Gemäß einer bevorzugten Ausführungsform des Verfahrens setzt sich die Laktobazillenflora des Impfgutes zu 50 bis 90 % aus Lb. pontis II DSM 14272 und/oder Lb. pontis III DSM 14273 und/oder Lb. pontis IV DSM 14274, zu 0,5 bis 5 % aus Lb. pontis I DSM 14269, zu 5 bis 20 % aus Lb. crispatus DSM 14271 und/oder Lb. plantarum DSM 14268 und zu 0,5 bis 5 % aus Lb. sanfranciscensis DSM 14270 zusammen. Eine Laktobazillenflora dieser Zusammensetzung ist sehr durchsetzungsfähig und setzt gleichzeitig eine ausgewogene Komposition von Säuren und Aromapräkursoren frei und ermöglicht so die Herstellung eines qualitativ hochwertigen Starters, der gleichbleibend gute Weizenvor-/ und Weizensauerteige hervorbringt.

Besonders zweckmäßig ist dabei eine adaptierte Mischflora, welche sowohl den vorstehend bezeichneten bevorzugten Hefestamm als auch die bevorzugte Laktobazillenflora enthält.

Zur reproduzierbaren Erzeugung des erfindungsgemäßen Starters ist es weiterhin zweckmäßig, wenn bei der Inokulierung des Getreidemahlprodukt-Wassergemisches mit dem Impfgut als Menge des Impfgutes 50 % (w/w) und mehr, vorzugsweise 80 % (w/w) und mehr und besonders bevorzugt zwischen 90 und 110 % (w/w) und insbesondere ca 100 % bezogen auf die Menge des verwendeten Getreidemahlproduktes eingesetzt werden. Besonders zweckmäßig ist auch, wenn das inokulierte Getreidemahlprodukt-Wassergemisch über wiederholte Zeitintervalle von ca. 8 bis 24, vorzugsweise zwischen 12 und 18 und besonders bevorzugt von ca. 16 Stunden kontinuierlich propagiert wird.

Es ist dabei zweckmäßig, wenn die Gesamtinkubationszeit (d.h. die Dauer der kontinuierlichen Propagation) in einzelne Zeitintervalle von 8-24 Stunden unterteilt ist und diese jeweils in eine Hoch- und Niedrigtemperaturphase unterteilt sind, wobei die Temperatur der Hochtemperaturphase bei zwischen 20 und 28°C, vorzugsweise bei zwischen 22 und 26°C, besonders bevorzugt bei ca. 24-26°C und die Temperatur während der niedrigen Temperaturphase bei zwischen 2 bis 16°C und vorzugsweise bei zwischen 4 und 12°C liegt. Es ist dabei besonders zweckmäßig, wenn Hoch- bzw. Niedrigtemperaturphasen jeweils ca. 50% der Dauer jedes Zeitintervalles ausmachen.

Zur Erzeugung eines besonders aktiven Weizenstarters ist es dabei besonders zweckmäßig, die Dauer der kontinuierlichen Propagation (d.h. die Gesamtinkubationszeit) so zu wählen, daß der Starter am Ende des Verfahrens eine adaptierte Mischflora mit 1 x 10⁸ bis 2 x 10⁹/g Laktobazillen oder, vorzugsweise und 5 x 10⁶ bis 5 x 10⁸/g Hefen enthält. Diese Keimdichte bringt gute Ergebnisse bei der Verwendung des hergestellten Starters zur Weizenvorteig- oder Weizensauerteigherstellung wie auch bei seiner Verwendung als Impfgut für die Herstellung von Startern. Es hat sich herausgestellt, daß zur Erzeugung eines besonders aktiven Starters eine Gesamt-Inkubationszeit von zwischen 5 bis 21 und vorzugsweise zwischen 7 bis 16, besonders bevorzugt 10 bis 14 Tagen zweckmäßig ist.

Je nach Art des mit dem erfindungsgemäß hergestellten Starters herzustellenden Weizenvor-/bzw. Sauerteiges kann es zweckmäßig sein, neben dem Weizenmahlprodukt ein oder mehrer weitere Getreidemahlprodukte für die Starterherstellung einzusetzen. Dies können weitere Weizenmahlprodukte, aber auch Mahlprodukte anderer Getreide sein. Für die Herstellung von Weizenvor-/bzw. Sauerteigen für Weizenmischbrote eignen sich beispielsweise besonders Starter, bei deren Herstellung außer Weizenmahlprodukten auch andere Getreidemahlprodukte eingesetzt werden. Die Wahl der jeweils best geeigneten Getreidemahlprodukte hängt unter anderem von der Art des herzustellenden Backproduktes ab und liegt im Kenntnisbereich des zuständigen Fachmanns.

Als Weizenmahlprodukte eignen sich besonders solche mit einem Aschegehalt von zwischen 550 bis 1050 mg pro 100g Mahlprodukt.

Bei Durchführung des erfindungsgemäßen Verfahrens bildet sich als Starterkultur eine adaptierte Mischflora mit einem von S. cerevisiae abgeleiteten Hefestamm Saccharomyces species DSM 14265 und einer Laktobazillenflora mit wenigstens drei der Laktobazillenstämme Lb. plantarum DSM 14268, Lb. pontis I DSM 14269, Lb. sanfraniscensis DSM 14270, Lb. crispatus DSM 14271, Lb. pontis II DSM 14272, Lb. pontis III DSM 14273, Lb. pontis IV DSM 14274 heraus.

Der so generierte Weizenstarter kann als Impfgut für die erneute Herstellung eines Weizenstarters eingesetzt werden oder wird zur Herstellung eines Weizenvorteiges oder Weizensauerteiges, insbesondere eines mild gesäuerten Weizensauerteiges weiterverwendet. Besonders vorteilhaft ist ein erfindungsgemäß hergestellter Starter mit einer adaptierten Mischflora mit dem von S. cerevisiae abgeleiteten Hefestamm Saccharomyces species DSM 14265 und einer Laktobaziilenflora mit 50 bis 90 % Lb. pontis Stämmen vorzugsweise bestehend aus Lb. pontis II DSM 14272 und/oder Lb. pontis III DSM 14273 und/oder Lb. pontis IV DSM 14274, mit 0,5 bis 5 % Lb. pontis I DSM 14269, 5 bis 20 % Lb. crispatus DSM 14271 und/oder Lb. plantarum DSM 14268 und 0,5 bis 5 % Lb. sanfranciscensis DSM 14270.

Das vorstehend beschriebe erfindungsgemäße Verfahren stellt eine Art "natürliches Reinzuchtverfahren im Lebensmittel" dar, bei welchem sich im Gegensatz zu den technologischen Reinzuchtverfahren (unter Sterilbedingungen, in artifiziellen Medien und meist in Monokultur) unter unsterilen Bäckereibedingungen im Teig selbst eine an das Vorteig- bzw. Sauerteig-Milieu angepaßte Mischkultur von speziellen Milchsäurebakterien und speziellen Hefen bildet. Der Einsatz dieser durch natürliche Reinzucht gewonnenen Mischkultur bzw. Starterkultur ermöglicht in Abhängigkeit der Fermentationsparameter (Temperatur, Stehzeit) die reproduzierbare Herstellung von qualitativ hochwertigen Weizenvorteigen und mild gesäuerten Weizensauerteigen in den Backbetrieben. Hiermit ist es für Bäckereien erstmals möglich, einen auf der Basis eines "natürliches Reinzuchtverfahren im Lebensmittel" hergestellten Weizenstarter zur indirekten Herstellung von Weizenbackwaren kommerziell zu beziehen.

Besonders zweckmäßig ist ein Verfahren, welches einen Starter hervorbringt, der selbst eine Mischflora mit einem von S. cerevisiae abgeleiteten Hefestamm Saccharomyces species DSM 14265 und einer Laktobazillenflora mit wenigstens einem der Laktobazillenstämme Lb. pontis I DSM 14269, Lb. pontis II DSM 14272, Lb. pontis III DSM 14273, Lb. pontis IV DSM 14274, Lb. crispatus DSM 14271, Lb. plantarum DSM 14268 und Lb. sanfranciscensis DSM 14270 und vorzugsweise eine adaptierte Mischflora mit dem von S. cerevisiae abgeleiteten Hefestamm Saccharomyces species DSM 14265 und einer Laktobazillenflora aufweist, die sich zu 50 bis 90 % aus Lb. pontis II DSM 14272 und/oder Lb. pontis III DSM 14273 und/oder Lb. pontis IV DSM 14274, zu 0,5 bis 5 % aus Lb. pontis I DSM 14269, zu 5 bis 20 % aus Lb. crispatus DSM 14271 und/oder Lb. plantarum DSM 14268, und zu 0,5 bis 5% Lb.sanfranciscensis DSM 14270 zusammensetzt.

Die erfindungsgemäße Herstellung von Startern und Starterkulturen durch "natürliche Reinzucht" mittels wiederholter Kultivierungs- und Rekultivierungszyklen im Lebensmittel bringt Kulturen hervor, die besonders gut an die Milieubedingungen der jeweiligen Sauerteig- oder Vorteigfermentation angepaßt sind.

Die Erfindung bezieht sich demgemäß auch auf die vorgenannten Mikroorganismen allein oder in beliebiger Kombination miteinander oder mit anderen Mikroorganismen sowie die vorstehend beschriebene adaptierte Mischflora. Die erfindungsgemäßen Mikroorganismen sind durch Anpassung an die jeweiligen Fermentationsbedingungen bestens für Starterkulturen/Anstellgüter für Weizenvorteige bzw. Weizensauerteige, insbesondere mild gesäuerte Weizensauerteige geeignet. Es hat sich gezeigt, daß wesentlicher Bestandteil der erfindungsgemäßen adaptierten Mikroflora Sauerteighefen und heterofermentative Laktobazillen sind, die an die empfohlenen Führungsweisen hervorragend adaptiert sind.

Die Erfindung bezieht sich überdies auf einen Starter, der mittels des erfindungsgemäßen Verfahrens herstellbar ist, sowie auf Starter mit den erfindungsgemäßen Mikroorganismen bzw. der erfindungsgemäßen adaptierten Mischflora.

Der erfindungsgemäße Starter und die darin enthaltene Starterkultur eignen sich für die Herstellung von Weizenvorteigen und mild gesäuerten Weizensauerteigen ebenso wie als Impfgut für die Herstellung neuer Sauerteigstarterkulturen für die Herstellung von Weizenvorteigen bzw. Weizensauerteigen.

Die erfindungsgemäße Starterkultur muß dabei nicht von dem umgebenden Medium getrennt werden, stattdessen kann der gesamte Starter direkt im Anschluß an seine Generierung zur Herstellung von Weizenvorteigen/Weizensauerteigen oder zur Herstellung neuer Starter eingesetzt werden.

Die Erfindung bezieht sich weiterhin auf ein Verfahren zur Herstellung von Weizenvor-/ und Weizensauerteigen, welches auf dem erfindungsgemäßen Starter bzw. den erfindungsgemäßen Mikroorganismen basiert. Bei diesem Verfahren wird einer zumindest ein Weizenmahlprodukt und Wasser enthaltenden Teigmasse der erfindungsgemäße Starter oder die erfindungsgemäße Starterkultur mit einem oder mehreren der erfindungsgemäßen Mikroorganismen oder (gemäß bäckerüblicher Führungsweise) ein damit hergestellter Weizenvor- oder Weizensauerteig als Anstellgut zugesetzt und zu einem Weizenvorteig oder Weizensauerteig, vorzugsweise einem Typ I Weizensauerteig mit milden Säuregraden umgesetzt. Diese Umsetzung wird im folgenden als Hauptprozess bezeichnet, im Gegensatz zur erfindungsgemäßen Herstellung des Starters, welche in diesem Zusammenhang den Vorprozess darstellt.

Zwar kann der erfindungsgemäß hergestellte Starter grundsätzlich für alle Verfahren zur Herstellung von Weizenvor-/ bzw. Weizensauerteigen eingesetzt werden, jedoch eignet er sich insbesondere für die Verwendung im erfindungsgemäßen Verfahren.

Die mikrobielle Stabilität des vergärenden inokulierten Getreidemahlprodukt-Wasser Gemisches wird im Bäckereibetrieb durch die Verwendung einer relativ hohen Menge an Anstellgut gewährleistet. So werden vorzugsweise ein Gewichtsteil des erfindungsgemäßen Starters, bzw. erfindungsgemäßen Weizenvorteiges bzw. Weizensauerteiges als Anstellgut zur Inokulierung von einem Gewichtsteil Getreidemahlprodukt in einem Volumenteil Wasser angesetzt. Gemäß eines bevorzugten Ausführungsbeispiels werden 15 kg Weizenvorteig, Weizensauerteig oder Starter als Anstellgut zur Inokulierung von 15 kg Getreidemahlprodukt in 15 I Wasser angesetzt.

Es ist dabei besonders zweckmäßig, wenn als Menge des Anstellgutes 50% und mehr, vorzugsweise 80% und mehr und besonders bevorzugt zwischen 90 und 110% und insbesondere ca. 100% der Menge des oder der verwendeten Getreidemahlprodukte eingesetzt werden. Der fertige Weizenvorteig bzw. Weizensauerteig kann dann vom Bäcker zur Backwarenherstellung eingesetzt werden. Ein Teil wird i.d.R. genutzt, um den Vorteig/Sauerteig bis zur nächsten Starterlieferung bäckerüblich weiterzuführen.

Ziel der Durchführung des erfindungsgemäßen Verfahrens zur Herstellung von Weizenvor-/bzw. Weizensauerteigen ist, daß das hergestellte Verfahrensprodukt (Weizenvor- bzw. Weizensauerteig) so stabil ist, daß sich die mikrobielle Flora der bäckerüblich geführten Weizenvorteig/Weizensauerteige innerhalb einer Woche bzw. eines Monates nicht verändert, so daß dem Bäcker bis zur nächsten Starterlieferung die Herstellung hochwertiger Weizenbackwaren ohne durch Fehlfermentation des Weizenvorteiges bzw. Weizensauerteiges bedingte Qualitätseinbußen möglich ist.

Zur Herstellung qualitativ besonders hochwertiger Weizenvor-/bzw. Sauerteige ist es besonders zweckmäßig, wenn die Gesamtdauer des Hauptprozesses so gewählt wird, daß der Anteil der heterofermentativen Laktobazillen an der Laktobazillenflora 50% und mehr, vorzugsweise 75% und mehr und besonders bevorzugt zwischen 80 und 90% ausmacht. Die prozentuale Verteilung der einzelnen Laktobazillenstämme kann dabei vom zuständigen Fachmann während der Verfahrensdurchführung auf bekannte Weise bestimmt werden.

Temperatur und/oder Gesamtdauer des Hauptprozesses können zweckmäßigerweise auch anhand des pH-Wertes des Verfahrensproduktes ausgerichtet sein, der nach Durchführung des Hauptprozesses für Weizenvorteige gleich oder größer als pH = 4,5 ist und vorzugsweise bei ca. pH = 4,8 bis 5,0 liegt und für Weizensauerteige kleiner als pH = 4,5 und vorzugsweise zwischen pH = 4,5 und pH = 4,2 (mild gesäuerte Weizensauerteige) liegt.

Die Aktivität der eingesetzten Sauerteigmikroorganismen sorgt dabei nicht nur für die gleichbleibend hohe Qualität der hergestellten Weizenvor- und Weizensauerteige, sondern trägt auch wesentlich zur Kürze des Hauptprozesses bei, was wiederum das Risiko der Kontamination mit Fremdkeimen verringert.

Es hat sich herausgestellt, daß zur Herstellung eines Weizenvorteiges bzw. mild gesäuerten Weizensauerteiges eine Gesamtdauer des Hauptprozesses von 8 bis 16 Stunden, vorzugsweise mit 8 Stunden bei 22 bis 26°C und 8 Stunden bei 4 bis 14 und vorzugsweise bei 4 bis 8°C und zur Herstellung eines mild gesäuerten Weizensauerteiges eine Gesamtdauer von 16 bis 24 Stunden, vorzugsweise mit 8 bis 16 Stunden bei 22 bis 26 °C und 16 bis 8 Stunden bei 4 bis 14 und vorzugsweise bei 4 bis 8°C zweckmäßig ist.

Es ist weiterhin zweckmäßig, wenn neben dem Weizenmahlprodukt ein oder mehrere weitere Getreidemahlprodukte für die Herstellung der Weizenvor-/bzw. mild gesäuerten Weizensauerteige verwendet werden. Dies können je nach Art der herzustellenden Backware andere Mahlprodukte anderer Getreide aber auch weitere Weizenmahlprodukte sowie Mischungen daraus sein. Als Weizenmahlprodukte sind für das erfindungsgemäße Verfahren insbesondere solche mit einem Aschegehalt von zwischen 550 bis 1050 mg pro 100 g Mahlprodukt geeignet.

Die Erfindung betrifft darüber hinaus ein Verfahren zur Herstellung von Weizenvor-/bzw. Weizensauerteig-Produkten (im folgenden zusammen als Convenience Produkte bezeichnet) zur direkten Herstellung von Backwaren auf Basis der erfindungsgemäßen Weizenvorteige oder Weizensauerteige mit Hilfe des oben genannten Weizenstarters oder der erfindungsgemäßen Starterkultur.

Bei diesem Verfahren schließt sich an das erfindungsgemäße Verfahren zur Herstellung von Weizenvor-/bzw. Weizensauerteigen eine Verfahrensstufe zur Stabilisierung des Verfahrensproduktes an. Bei der Stabilisierung werden die Mikroorganismen nicht abgetötet, ihr Stoffwechsel und ihre Vermehrung durch geeignete Mapnahmen jedoch so weit wie möglich gehemmt bzw. unterbunden.

Die so hergestellten Convenience-Produkte können in flüssiger, pastöser sowie granulatartiger/pulvriger Form vorliegen. Hierbei werden die erfindungsgemäß hergestellten Weizenvorteige bzw. Weizensauerteige nach der Herstellung stabilisiert, so daß sie beim Anwender (Bäcker) auch nach längerem Transport oder Lagerung nicht mehr stoffwechselaktiv sind und ohne Qualitätseinbußen des Endproduktes für die Verwendung in einer direkten Herstellungsweise eingesetzt werden können.

Die Stabilisierung kann grundsätzlich durch jede Maßnahme erfolgen, die geeignet ist, die Mikroflora vor Kontaminationen zu schützen und das Wachstum und den Stoffwechsel der Mikroflora zu unterbinden, so daß das Produkt stabil bleibt und sich für den Einsatz bei der direkten Brotteigherstellung eignet. Gemäß einer bevorzugten Ausführungsform erfolgt die Stabilisierung durch Zugabe von Essigsäure oder Essig und/oder Salz oder Essig/Essigsäure, Salz und Mehl, vorzugsweise die Zugabe von Essig (insbesondere Lebensmittelessig mit einer Essigsäurekonzentration von 8 bis 12, vorzugsweise ca. 10%) zu einer Endkonzentration von 5 -15% (w/w) und/oder Kochsalz (NaCI) zu einer Endkonzentration von 2-10% (w/w) und/oder Weizenmehl auf eine Endkonzentration von 10-50% (w/w), jeweils bezogen auf den Weizensauerteig bzw. Weizenvorteig. Diese Stabilisierungsmaßnahmen einzeln oder im Verbund sind wirksam und entsprechen gleichzeitig den Anforderungen an eine ökologische Backwarenherstellung, da es sich bei den zugesetzten Stoffen um traditionelle Lebensmittelzutaten handelt, die für den menschlichen Organismus unschädlich sind.

Mit Hilfe der so hergestellten Convenience Produkte ist der Anwender (Bäcker) in der Lage auch ohne einen selbstgeführten Betriebsauerteig bzw. Vorteig Weizengebäckqualitäten herzustellen, die denen einer indirekten Herstellungsweise ebenbürtig sind. Der Vorteil für den Anwender liegt hierbei in der Einsparung von Zeit, Investitionskosten für Anlagen, flexibler Produktion, einer konstant guten Qualität und der Möglichkeit auch mit wenig erfahrenem Personal gute Gebäckqualitäten zu erzielen.

Die Erfindung bezieht sich schließlich auf die erfindungsgemäßen Convenience-Produkte sowie deren Verwendung bei der direkten Herstellung von Backwaren, insbesondere Weizenbackwaren.

Unter dem Begriff "Convenience-Produkt" wird das gesamte, durch das vorstehend beschriebene Verfahren gewonnene Verfahrensprodukt verstanden, welches unter anderem die noch nicht, teil- oder vollvergorenen Substrate (das oder die Getreidemahlprodukte), die von der mikrobiellen Flora erzeugten Aromapräkursoren sowie die zur mikrobiellen Stabilisierung der Produkte zugesetzten Substanzen (Essig/Essigsäure und/oder Salz und/oder Mehl) enthält.

Die Erfindung wird nachfolgend anhand von bevorzugten Ausführungsbeispielen näher erläutert, welche sich auf die direkte Backwarenherstellung mit Hilfe eines erfindungsgemäßen Weizensauerteig-Produktes beziehen.

### Beispiel 1: Rezeptur für Weizenbrot:

| | |
|---|---|
| | 100 % Weizenmehl |
| + | 5 bis 20% (w/w) Weizensauerteig-Produkt (bezogen auf Weizenmehl) |
| + ca. | 60 % (w/w) Wasser (bezogen auf Weizenmehl) |
| + | 1,5-0,4% (w/w) Salz (bezogen auf Weizenmehl) |
| + | 3% (w/w) Hefe (bezogen auf Weizenmehl) |

Das in diesem Beispiel verwendete Weizensauerteig-Produkt wurde durch Zugabe von 10g Essig (10% ig) und die Zugabe von 7,5 g NaCI pro 100g Weizensauerteig stabilisiert, der Weizensauerteig selbst wurde mit Hilfe der erfindungsgemäßen Mischkultur hergestellt. Da Stoffwechsel und Vermehrung der mikrobiellen Flora im Weizensauerteig-Produkt unterbunden sind, kann diese nicht zur Teighebung beitragen. Die Teighebung wird daher durch die zugesetzte Backhefe bewirkt (direkte Backwarenherstellung).

Der so im Bäckereibetrieb "direkt hergestellte" Weizenbrotteig sollte wie folgt verarbeitet werden:

| | |
|---|---|
| Teigknetung: | Hubkneter 1+5 Minuten |
| | Spiralkneter 1+4 Minuten |
| Teigausbeute: | ca. 160 |
| Teigtemparatur: | 26°C |
| Teigruhe: | 15 Minuten |

Nach der Teigruhe Teiglinge abwiegen, rundstoßen und nochmals 15 Minuten ruhen lassen.

| | |
|---|---|
| Stückgare: | 30-40 Minuten |
| Backtemperatur: | 230 °C |
| Backzeit: | 30-40 Minuten (für 500 g Brote) |

Diese Rezeptur ist anwendbar für alle Weizengebäcke wie z.B. Brötchen, Weißbrot, Baguette, Ciabatta oder feine Backwaren.

## Patentansprüche

1. Verfahren zur Herstellung eines Starters für die Herstellung von Weizenvorteigen und Weizensauerteigen unter Verwendung von Wasser und mindestens eines Weizenmahlproduktes, welches durch Zusatz eines Laktobazillen und Hefen enthaltenden Impfgutes teilweise vergoren wird
**dadurch gekennzeichnet,**
**daß** das Impfgut eine adaptierte Mischflora mit mindestens einem Hefestamm und mit einer Laktobazillenflora mit mindestens jeweils einem Stamm homo- und heterofermentativer Laktobazillen enthält und bei der Inokulierung des Getreidemahlprodukt-Wasser Gemisches mit dem Impfgut als Menge des Impfgutes 50 % (w/w) und mehr, vorzugsweise 80 % (w/w) und mehr und besonderes bevorzugt zwischen 90 und 110 % (w/w) und insbesondere ca. 100 % (w/w) der Menge des oder der verwendeten Getreidemahlprodukte eingesetzt werden, wobei die adaptierte Mischflora eine an die Kulturbedingungen im vergärenden Getreidemahlprodukt-Wasser-Gemisch angepasste Mikroorganismenflora ist, welche sich bei fortwährender Kokultivierung der Mikroorganismen im Gemisch durch wiederholte Kultivierungs- und Rekultivierungszyklen herausbildet.

2. Verfahren gemäß Anspruch 1, **dadurch gekennzeichnet, daß** als Hefe ein von S. cerevisiae abgeleiteter Sauerteigstamm und vorzugsweise der Stamm Saccharomyces species DSM 14265 in der adaptierten Mischflora des Impfgutes enthalten ist.

3. Verfahren gemäß einem der vorstehenden Ansprüche 1 oder 2, **dadurch gekennzeichnet, daß** die Laktobazillenflora des Impfgutes wenigstens drei der Laktobazillenstämme Lb. plantarum DSM 14268, Lb. pontis I DSM 14269, Lb. sanfranciscensis DSM 14270, Lb. crispatus DSM 14271, Lb. pontis II DSM 14272, Lb. pontis III DSM 14273, Lb. pontis IV DSM 14274 enthält.

4. Verfahren gemäß Anspruch 3, **dadurch gekennzeichnet, daß** sich die Laktobazillenflora zu 50 bis 90 % aus Lb. pontis II DSM 14272 und/oder Lb. pontis III DSM 14273 und/oder Lb. pontis IV DSM 14274, zu 0,5 bis 5 % aus Lb. pontis I DSM 14269, zu 5 bis 20 % aus Lb. crispatus DSM 14271 und/oder Lb. plantarum DSM 14268 und zu 0,5 bis 5 % aus Lb. sanfranciscensis DSM 14270 zusammensetzt.

5. Verfahren gemäß einem der vorstehenden Ansprüche 1 bis 4, **gekennzeichnet durch** die kontinuierliche Propagation über wiederholte Zeitintervalle von jeweils ca. 8 bis 24, vorzugsweise zwischen 12 bis 18 und besonders bevorzugt von ca. 16 Stunden.

6. Verfahren gemäß Anspruch 5, **dadurch gekennzeichnet, daß** die Dauer der kontinuierlichen Propagation so gewählt wird, daß der Starter eine adaptierte Mischflora mit 1 x 10⁸ bis 2 x 10^{/9}/g Laktobazillen und 5 x 10⁶ bis 5 x 10⁸/g Hefen enthält.

7. Verfahren gemäß einem der Ansprüche 5 oder 6, **dadurch gekennzeichnet, daß** die Gesamtdauer der kontinuierlichen Propagation in ca. 8 bis 24 Stunden dauernden Zeitintervallen zwischen 5 bis 21, vorzugsweise zwischen 7 bis 16 und besonders bevorzugt zwischen 12 bis 14 Tage beträgt.

8. Verfahren gemäß einem der Ansprüche 5 bis 7, **dadurch gekennzeichnet, daß** jedes Zeitintervall in eine Hoch- und eine Niedrigtemperaturphase unterteilt ist, wobei die Temperatur der Hochtemperaturphase bei zwischen 20 und 28°C, vorzugsweise bei zwischen 22 und 26°C und besonders bevorzugt bei ca 24 bis 26°C, und die Temperaturwerte der Niedrigtemperaturphase bei zwischen 2 bis 16°C und vorzugsweise zwischen 4 und 12°C liegen.

9. Verfahren gemäß einem der vorstehenden Ansprüche 1 bis 8, **dadurch gekennzeichnet, daß** neben dem Weizenmahlprodukt ein oder mehrere weitere Getreidemahlprodukte eingesetzt werden.

10. Verfahren gemäß Anspruch 9, **dadurch gekennzeichnet, daß** das oder die Weizenmahlprodukte einen Aschegehalt von zwischen 550 bis 1050 mg pro 100 g Mahlprodukt aufweisen.

11. Verfahren gemäß einem der Ansprüche 1 bis 10, **dadurch gekennzeichnet, daß** der generierte Starter eine adaptierte Mischflora mit einem von S. cerevisiae abgeleiteten Hefestamm Saccharomyces species DSM 14265 und einer Laktobazillenflora mit wenigstens drei der Laktobazillenstämme Lb. plantarum DSM 14268, Lb. pontis I DSM 14269, Lb. sanfranciscensis DSM 14270, Lb. crispatus DSM 14271, Lb. pontis II DSM 14272, Lb. pontis III DSM 14273, Lb. pontis IV DSM 14274 und vorzugsweise mit einer adaptierten Mischflora mit dem von S. cerevisiae abgeleiteten Hefestamm Saccharomyces species DSM 14265 und einer Laktobazillenflora mit 50 bis 90 % Lb. pontis Stämmen, vorzugsweise Lb. pontis II DSM 14272, und/oder Lb. pontis III DSM 14273, und/oder Lb. pontis IV DSM 14274, mit 0,5 bis 5 % Lb. pontis I DSM 14269, 5 bis 20 % Lb. crispatus DSM 14271 und/oder Lb. plantarum DSM 14268 und 0,5 bis 5 % Lb. sanfranciscensis DSM 14270 enthält.

12. Stämme der Spezies Saccharomyces species DSM 14265, Lb. pontis I DSM 14269, Lb. pontis II DSM 14272, Lb. pontis III DSM 14273, Lb. pontis IV DSM 14274, Lb. crispatus DSM 14271, Lb. plantarum DSM 14268 und Lb. sanfranciscensis DSM 14270 allein oder in beliebiger Kombination miteinander oder mit anderen Mikroorganismen.

13. Adaptierte Mischflora gemäß Anspruch 11.

14. Starter, erhältlich gemäß einem der vorstehenden Ansprüche 1 bis 11.

15. Starter mit einem oder mehreren der Stämme gemäß Anspruch 12 und vorzugsweise mit einer adaptierten Mischflora gemäß Anspruch 13.

16. Verwendung eines oder mehrer Stämme gemäß Anspruch 12 oder einer adaptierten Mischflora gemäß Anspruch 13 als Impfgut für die Herstellung von Startern oder Starterkulturen oder als Starterkultur für die Herstellung von Weizenvor- oder mild gesäuerten Weizensauerteigen.

17. Verwendung eines Starters gemäß einem der Ansprüche 14 oder 15 zur Herstellung von Weizenvor- und Weizensauerteigen, vorzugsweise mild gesäuerten. Weizensauerteigen und als Impfgut für die Herstellung von Startern und Starterkulturen.

18. Verfahren zur Herstellung eines Weizenvorteiges oder Weizensauerteiges, **dadurch gekennzeichnet, daß** einem zumindest ein Weizenmahlprodukt und Wasser enthaltenden Gemisch ein Starter gemäß Anspruch 14 oder 15 oder eine Starterkultur mit einem oder mehreren Mikroorganismen gemäß Anspruch 12 oder einer adaptierten Mischflora gemäß Anspruch 13 oder ein mit dem Starter oder der Starterkultur oder der Mischflora hergestellter Weizenvor- oder Weizensauerteig als Anstellgut zugesetzt und in mindestens einer Verfahrensstufe (Hauptprozeß) zu einem Weizenvorteig oder Weizensauerteig, vorzugsweise einem Typ I Weizensauerteig mit milden Säuregraden umgesetzt wird.

19. Verfahren gemäß Anspruch 18, **dadurch gekennzeichnet, daß** als Menge des Anstellgutes 50 % und mehr, vorzugsweise 80 % und mehr und besonders bevorzugt zwischen 90 und 110 % und insbesondere ca. 100 % der Menge des oder der verwendeten Getreidemahlprodukte eingesetzt werden.

20. Verfahren gemäß einem der Ansprüche 18 oder 19, **dadurch gekennzeichnet, daß** die Gesamtdauer des Hauptprozesses so gewählt wird, daß der Anteil der heterofermentativen Laktobazillen an der Laktobazittenflora am Ende des Hauptprozesses 50 % und mehr, vorzugsweise 75 % und mehr und besonders bevorzugt zwischen 80 und 90 % ausmacht.

21. Verfahren gemäß einem der Ansprüche 19 bis 21, **dadurch gekennzeichnet, daß** die Dauer des Hauptprozesses so gewählt wird, daß der pH-Wert des Verfahrensproduktes nach Durchführung des Hauptprozesses gleich oder größer ist als pH= 4,5 und vorzugsweise bei ca. pH = 5,0 liegt.

22. Verfahren gemäß einem der Ansprüche 18 bis 20, **dadurch gekennzeichnet, daß** die Dauer des Hauptprozesses so gewählt wird, daß der pH-Wert des Verfahrensproduktes nach Durchführung des Hauptprozesses kleiner ist als pH= 4,5 und vorzugsweise bei ca. pH = 4,2 bis 4,5 liegt.

23. Verfahren gemäß einem der Ansprüche 18 bis 22, **dadurch gekennzeichnet, daß** die Dauer des Hauptprozesses zur Herstellung eines Weizenvorteiges zwischen 8 - 16 Stunden, vorzugsweise 8 Stunden bei 22-26°C und 8 Stunden bei 4 bis 14, vorzugsweise 4 bis 8°C, und zur Herstellung eines mild gesäuerten Weizensauerteiges zwischen 16-24 Stunden, vorzugsweise 8-16 Stunden bei 22-26°C und 16-8 Stunden bei 4 bis 14, vorzugsweise 4-8°C beträgt.

24. Verfahren gemäß einem der Ansprüche 18 bis 23, **dadurch gekennzeichnet, daß** der Hauptprozeß zur Herstellung eines Weizenvorteiges bzw. mild gesäuerten Weizensauerteiges bei Temperaturen zwischen 21 und 27°C, vorzugsweise zwischen 22 und 26°C und besonders bevorzugt bei ca. 24°C umfaßt.

25. Verfahren gemäß einem der vorstehenden Ansprüche 18 bis 24 **dadurch gekennzeichnet, daß** neben dem Weizenmahlprodukt ein oder mehrere weitere Getreidemahlprodukte eingesetzt werden.

26. Verfahren gemäß Anspruch 25, **dadurch gekennzeichnet, daß** das oder die Weizenmahlprodukte einen Aschegehalt von zwischen 550 bis 1050 mg pro 100 g Mahlprodukt aufweisen.

27. Verfahren gemäß einem der vorstehenden Ansprüche 18 bis 25, **dadurch gekennzeichnet, daß** sich an den Hauptprozeß eine Verfahrensstufe zur Stabilisierung des Verfahrensproduktes anschließt.

28. Verfahren gemäß Anspruch 27, **dadurch gekennzeichnet, daß** die Stabilisierung des Convenienceproduktes durch die Zugabe von Essigsäure oder Essig und/oder Salz und/oder Mehl, insbesondere durch die Zugabe von Essig zu einer Endkonzentration von 5-15% (w/w) und/oder Kochsalz (NaCl) zu einer Endkonzentration von 2-10% (w/w) und/oder Mehl zu einer Endkonzentration von 10-50% bezogen auf Weizensauerteig bzw. Weizerivorteig erfolgt.

29. Weizenvorteig- oder Weizensauerteig-Produkt erhältlich durch das Verfahren nach Anspruch 28.

30. Verwendung eines Weizensauerteig-Produktes gemäß Anspruch 30 bei der Herstellung von Backwaren insbesondere von Weizenbackwaren.

## Claims

1. Method of product a starter for the production of wheat predoughs and wheat sourdoughs using water and at least one milled wheat product which is fermented by the addition of an inoculant containing lactobacilli and yeasts, **characterised in that** the inoculant contains an adapted mixed flora with at least one yeast strain and with a lactobacillus flora with at least in each case a strain of homo- and heterofermentative lactobacilli and duration the inoculation of the mixture of milled wheat product and water with the inoculant 50% (w/w) and more, preferably 80% (w/w) and more particularly preferably between 90 and 110% (w/w) and particularly approximately 100% (w/w) of the amount of the milled grain product or products used are used as the amount of inoculant, wherein the adapted mixed flora is a microorganism flora which is adapted to the culture conditions in the fermenting mixture of milled grain product and water and which develops during continuous cocultivation of the microorganisms in the mixture by repeated cultivation and recultivation cycles.

2. Method as claimed in Claim 1, **characterised in that** a sourdough stain derived from S. cerevisiae and preferably the strain Saccharomyces species DSM 14265 is contained as yeast in the adapted mixed flora of the inoculant.

3. Method as claimed in any one of Claims 1 or 2, **characterised in that** the lactobacillus flora of the inoculant contains at least three of the lactobacillus strains Lb. plantarum DSM 14268, Lb. pontis I DSM 14269, Lb. sanfranciscensis DSM 14270, Lb. crispatus DSM 14271, Lb. pontis II DSM 14272, Lb. pontis III DSM 14273, Lb. pontis IV DSM 14274.

4. Method as claimed in Claim 3, **characterised in that** of the lactobacillus flora 50 to 90% is composed of Lb. pontis II DSM 14272 and/or Lb. pontis III DSM 14273 and/or Lb. pontis IV DSM 14274, 0.5 to 5% is composed of Lb. pontis I DSM 14269, 5 to 20% is composed of Lb. crispatus DSM 14271 and/or Lb. plantarum DSM 14268 and 0.5 to 5% is composed of Lb. sanfranciscensis DSM 14270.

5. Method as claimed in any one of the preceding Claims 1 to 4, **characterised by** the continuous propagation over repeated time intervals of in each case approximately 8 to 24 hours, preferably between 12 to 18 hours, particularly preferably approximately 16 hours.

6. Method as claimed in Claim 5, **characterised in that** the duration of the continuous propagation is chosen so that the starter contains an adapted mixed flora with 1 x 10⁸ to 2 x 10⁹/g lactobacilli and 5 x10⁶ to 5 x 10⁸/g yeasts.

7. Method as claimed in any one of Claims 5 or 6, **characterised in that** the total duration of the continuous propagation in time intervals lasting approximately 8 to 14 hours is between 5 and 21 days, preferably between 7 and 16 days and particularly preferably between 12 and 14 days.

8. Method as claimed in any one of Claims 5 to 7, **characterised in that** each time interval is divided into a high-temperature phase and a low-temperature phase, wherein the temperature of the high-temperature phase is between 20 and 28°C, preferably between 22 and 26°C and particularly preferably approximately 24 to 26°C, and the temperature values of the low-temperature phase are between 2 and 16°C and preferably between 4 and 12°C.

9. Method as claimed in any one of the preceding Claims 1 to 8, **characterised in that** in addition to the milled wheat product one or more further milled grain products are used.

10. Method as claimed in Claim 9, **characterised in that** the milled wheat product or products have an ash content of between 550 and 1050 mg per 100 g of milled product.

11. Method as claimed in any one of Claims 1 to 10, **characterised in that** the generated starter contains an adapted mixed flora with a yeast strain derived from S. cerevisiae, Saccharomyces species DSM 14265, and a lactobacillus flora with at least three of the lactobacillus strains Lb. plantarum DSM 14268, Lb. pontis I DSM 14269, Lb. sanfranciscensis DSM 14270, Lb. crispatus DSM 14271, Lb. pontis II DSM 14272, Lb. pontis III DSM 14273, Lb. pontis IV DSM 14274 and preferably with an adapted mixed flora with a yeast strain derived from S. cerevisiae, Saccharomyces species DSM 14265 and a lactobacillus flora with 50 to 90% Lb. pontis strains, preferably Lb. pontis II DSM 14272 and/or Lb. pontis III DSM 14273 and/or Lb. pontis IV DSM 14274, with 0.5 to 5% Lb. pontis I DSM 14269, 5 to 20% Lb. crispatus DSM 14271 and/or Lb. plantarum DSM 14268 and 0.5 to 5% Lb. sanfranciscensis DSM 14270.

12. Strains of the species Saccharomyces species DSM 14265, Lb. pontis I DSM 14269, Lb. pontis II DSM 14272, Lb. pontis III DSM 14273, Lb. pontis IV DSM 14274, Lb. crispatus DSM 14271, Lb. plantarum DSM 14268 and Lb. sanfranciscensis DSM 14270 alone or in any combination with one another or with other microorganisms.

13. Adapted mixed flora as claimed in Claim 11.

14. Starter obtainable as claimed in any one of the preceding Claims 1 to 11.

15. Starter with one or several of the strains as claimed in claim 12 and preferably with an adapted mixed flora as claimed in Claim 13.

16. Use of one or several stains as claimed in Claim 12 or an adapted mixed flora as claimed in Claim 13 as inoculant for the production of starters or starter culture for the production of wheat predoughs or mildly soured wheat sourdough.

17. Use of a starter as claimed in any one of Claims 14 or 15 for the production of wheat predoughs and wheat sourdoughs, preferably mildly soured wheat sourdoughs and as inoculant for the production of starters and starter cultures.

18. Method for the production of a wheat predough or wheat sourdough, **characterised in that** a starter as claimed in Claim 14 or 15 or a starter culture with one or several microorganisms as claimed in Claim 12 or an adapted mixed flora as claimed in Claim 13 or a wheat predough or wheat sourdough produced with the starter or the starter culture or the mixed flora is added as starter material to a mixture containing at least one milled wheat product and water, and is converted in at least one method stage (main process) to a wheat predough or wheat sourdough, preferably a type I wheat sourdough with mild degrees of acidity.

19. Method as claimed in Claim 18, **characterised in that** 50% and more, preferably 80% and more and particularly preferably between 90 and 110% and in particular approximately 100% of the amount of the milled grain product or products used are used as the amount of starter material.

20. Method as claimed in any one of Claims 18 or 19, **characterised in that** the total duration of the main process is chosen so that the proportion of the heterofermentative lactobacilli in the lactobacillus flora at the end of the main process makes up 50% and more, preferably 75% and more and particularly preferably between 80 and 90%.

21. Method as claimed in any one of Claims 19 to 21, **characterised in that** the duration of the main process is chosen so that the pH value of the product of the method after the main process has been carried out is equal to or greater than pH = 4.5 and preferably approximately pH=5.0.

22. Method as claimed in any one of Claims 18 to 20, **characterised in that** the duration of the main process is chosen so that the pH value of the product of the method after the main process has been carried out is less than pH = 4.5 and preferably approximately pH = 4.2 to 4.5.

23. Method as claimed in any one of Claims 18 to 22, **characterised in that** the duration of the main process for the production of a wheat predough is between 8 to 16 hours, preferably 8 hours at 22 to 26°C and 8 hours at 4 to 14°C, preferably 4 to 8°C, and for the production of a mildly soured wheat sourdough between 16 and 24 hours, preferably 8 to 16 hours at 22 to 26°C and 16 to 8 hours at 4 to 14°C, preferably 4 to 8°C.

24. Method as claimed in any one of Claims 18 to 23, **characterised in that** the main process for the production of a wheat predough or mildly soured wheat sourdough comprises temperatures between 21 and 27°C, preferably between 22 and 26°C and particularly preferably approximately 24°C.

25. Method as claimed in any one of the preceding Claims 18 to 24, **characterised in that** in addition to the milled wheat product one or several further milled grain products are used.

26. Method as claimed in Claim 25, **characterised in that** the milled heat product or products have an ash content of between 550 and 1050 mg per 100 g of milled product.

27. Method as claimed in any one of the preceding Claims 18 to 25, **characterised in that** a method stage for the stabilisation of the product of the method follows the main process.

28. Method as claimed in Claim 27, **characterised in that** the stabilisation of the convenience product takes place by the addition of acetic acid or vinegar and/or salt and/or flour, in particular by the addition of vinegar to an end concentration of 5 to 15% (w/w) and/or common salt (NaCl) to an end concentration of 2 to 10% (w/w) and/or flour to an end concentration of 10 to 50% based on wheat sourdough or wheat predough.

29. Wheat predough or wheat sourdough product obtainable by the method as claimed in Claim 28.

30. Use of a wheat sourdough product as claimed in Claim 30 in the production of bakery products, particularly wheat bakery products.

## Revendications

1. Procédé pour la préparation d'un starter pour la préparation de levains-chefs et de levains de panification à base de froment, reposant sur l'utilisation d'eau et d'au moins un produit de type mouture de froment, lequel est partiellement fermenté en ajoutant un inoculum contenant des lactobacilles et des levures, **caractérisé en ce que** l'inoculum contient une flore mixte adaptée présentant au moins une souche de levure et une flore de lactobacilles contenant à chaque fois au moins une souche de lactobacilles homo- et hétérofermentaires et **en ce que** l'on utilise, lors de l'inoculation du mélange eau-produit de type mouture de céréale avec l'inoculum, en tant que quantité d'inoculum 50 % (p/p) et plus, de préférence 80 % et plus (p/p), de manière particulièrement préférée entre 90 et 110 % (p/p) et de manière préférée entre toutes environ 100 % (p/p) de la quantité du ou des produits de type mouture de céréale utilisée, la flore mixte adaptée étant une flore de micro-organismes adaptée aux conditions de culture dans un mélange eau-produit de type mouture de céréale en fermentation, laquelle se forme lors de la co-culture continue des micro-organismes dans le mélange au cours de cycles répétés de culture et de reculture.

2. Procédé selon la revendication 1, **caractérisé en ce qu'**une souche de levain de panification dérivée du S. cerevisiae, et de préférence la souche de l'espèce Saccharomyces DSM 14265, est présente, en tant que levure, dans la flore mixte adaptée de l'inoculum.

3. Procédé selon l'une quelconque des revendications précédentes 1 ou 2, **caractérisé en ce que** la flore de lactobacilles de l'inoculum contient au moins trois des souches de lactobacilles Lb. plantarum DSM 14268, Lb. pontis I DSM 14269, Lb. sanfranciscensis DSM 14270, Lb. crispatus DSM 14271, Lb. pontis II DSM 14272, Lb. pontis III DSM 14273, Lb. pontis IV DSM 14274.

4. Procédé selon la revendication 3, **caractérisé en ce que** la flore de lactobacilles se compose de 50 à 90 % de Lb. pontis II DSM 14272 et/ou Lb. pontis III DSM 14273 et/ou Lb. pontis IV DSM 14274, de 0,5 à 5 % de Lb. pontis I DSM 14269, de 5 à 20 % de Lb. crispatus DSM 14271 et/ou Lb. plantarum DSM 14268 et de 0,5 à 5 % de Lb. sanfranciscensis DSM 14270.

5. Procédé selon l'une quelconque des revendications précédentes 1 à 4, **caractérisé par** la propagation en continu dans des intervalles de temps répétitifs d'environ 8 à 24 heures, de préférence d'environ 12 à 18 heures et de manière particulièrement préférée d'environ 16 heures.

6. Procédé selon la revendication 5, **caractérisé en ce que** la durée de la propagation en continu est sélectionnée, de sorte que le starter contient une flore mixte adaptée présentant de 1 x 10⁸ à 2 x 10⁹/g de lactobacilles et de 5 x 10⁶ à 5 x 10⁸/g de levures.

7. Procédé selon l'une quelconque des revendications 5 ou 6, **caractérisé en ce que** la durée totale de la propagation en continu s'inscrit entre environ 8 et 24 heures dans des intervalles durant de 5 à 21 jours, de préférence de 7 à 16 jours et de manière particulièrement préférée de 12 à 14 jours.

8. Procédé selon l'une quelconque des revendications 5 à 7, **caractérisé en ce que** chaque intervalle de temps est décomposé en une phase à haute température et en une phase à basse température, les températures de la phase à haute température s'inscrivant entre 20 et 28°C, de préférence entre 22 et 26°C et de manière particulièrement préférée entre environ 24 et 26°C, et les températures de la phase à basse température s'inscrivant entre 2 et 16°C et de préférence entre 4 et 12°C.

9. Procédé selon l'une quelconque des revendications précédentes 1 à 8, **caractérisé en ce que** parallèlement au produit de type mouture de froment, on utilise un ou plusieurs autres produits de type mouture de céréale.

10. Procédé selon la revendication 9, **caractérisé en ce que** le ou les produits de type mouture de froment présentent une teneur en cendres s'inscrivant entre 550 et 1 050 mg par 100 g de produit de type mouture.

11. Procédé selon l'une quelconque des revendications 1 à 10, **caractérisé en ce que** le starter généré contient une flore mixte adaptée présentant une souche de levures dérivée de la S. cerevisiae, espèce Saccharomyces DSM 14265 et une flore de lactobacilles présentant au moins trois des souches de lactobacilles Lb. plantarum DSM 14268, Lb. pontis I DSM 14269, Lb. sanfranciscensis DSM 14270, Lb. crispatus DSM 14271, Lb. pontis II DSM 14272, Lb. pontis III DSM 14273, Lb. pontis IV DSM 14274 et de préférence, une flore mixte adaptée présentant une souche de levures dérivée de la S. cerevisiae, espèce Saccharomyces DSM 14265 et une flore de lactobacilles comprenant de 50 à 90 % de souches de LB. pontis, de préférence Lb. pontis II DSM 14272, et/ou Lb. pontis III DSM 14273, et/ou Lb. pontis IV DSM 14274, de 0,5 à 5 % de Lb. pontis I DSM 14269, de 5 à 20 % de Lb. crispatus DSM 14271 et/ou Lb. plantarum DSM 14268 et de 0,5 à 5 % de Lb. sanfranciscensis DSM 14270.

12. Souches des espèces Saccharomyces DSM 14265, Lb. pontis I DSM 14269, Lb. pontis II DSM 14272, Lb. pontis III DSM 14273, Lb. pontis IV DSM 14274, Lb. crispatus DSM 14271, Lb. plantarum DSM 14268 et Lb. sanfranciscensis DSM 14270 seules ou de préférence combinées les unes avec les autres ou avec d'autres micro-organismes.

13. Flore mixte adaptée selon la revendication 11.

14. Starter disponible selon l'une quelconque des revendications précédentes 1 à 11.

15. Starter contenant une ou plusieurs des souches selon la revendication 12 et de préférence comprenant une flore mixte adaptée selon la revendication 13.

16. Utilisation d'une ou plusieurs souches selon la revendication 12 ou d'une flore mixte adaptée selon la revendication 13 en tant qu'inoculum pour la fabrication de starters et de cultures de starters ou en tant que culture de starters pour la préparation de levains-chefs à base de froment ou de levains de panification légèrement acidifiés à base de froment.

17. Utilisation d'un starter selon l'une quelconque des revendications 14 ou 15, pour la préparation de levains-chefs à base de froment ou de levains de panification à base de froment, de préférence de levains de panification légèrement acidifiés à base de froment, et en tant qu'inoculum pour la fabrication de starters et de cultures de starters.

18. Procédé pour la préparation d'un levain-chef ou d'un levain de panification à base de froment, **caractérisé en ce qu'**un starter selon la revendication 14 ou 15 ou une culture de starters présentant un ou plusieurs micro-organismes selon la revendication 12 ou une flore mixte adaptée selon la revendication 13 ou un levain-chef ou un levain de panification à base de froment préparé à l'aide du starter ou de la culture de starters ou de la flore mixte est ajouté à un mélange contenant au moins un produit de type mouture de froment et de l'eau en tant que produit d'amorçage et est converti au moins au cours d'une étape du procédé (procédé principal) en un levain-chef à base de froment ou en un levain de panification à base de froment, de préférence en un levain de panification de type 1 présentant de légers degrés acides.

19. Procédé selon la revendication 18, **caractérisé en ce qu'**on utilise, en tant que quantité de produit d'amorçage, 50 % et plus, de préférence 80 % et plus, et de manière particulièrement préférée entre 90 et 110 % et de manière préférée entre toutes environ 100 % de la quantité du ou des produits de type mouture de céréale.

20. Procédé selon l'une quelconque des revendications 18 ou 19, **caractérisé en ce que** la durée totale du procédé principal est choisie de sorte que la proportion de lactobacilles hétérofermentaires rapportée à la flore de lactobacilles représente à la fin du procédé principal 50 % et plus, de préférence 75 % et plus et de manière particulièrement préférée entre 80 et 90 %.

21. Procédé selon l'une quelconque des revendications 19 à 21, **caractérisé en ce que** la durée du procédé principal est choisie de sorte que la valeur de pH du produit du procédé après réalisation du procédé principal soit supérieure ou égale à un pH = 4,5 et s'élève de préférence à un pH = 5,0 environ.

22. Procédé selon l'une quelconque des revendications 18 à 20, **caractérisé en ce que** la durée du procédé principal est choisie de sorte que la valeur de pH du produit du procédé après réalisation du procédé principal soit inférieure à un pH = 4,5 et qu'elle se situe de préférence dans une plage de pH de 4,2 à 4,5.

23. Procédé selon l'une quelconque des revendications 18 à 22, **caractérisé en ce que** la durée du procédé principal pour la préparation d'un levain-chef à base de froment s'inscrit entre 8 et 16 heures, de préférence de 8 heures entre 22 et 26°C et 8 heures entre 4 et 14°C, de préférence 4 à 8°C, et pour la préparation d'un levain de panification légèrement acidifié à base de froment entre 16 et 24 heures, de préférence 8 à 16 heures entre 22 et 26°C et 16 à 8 heures entre 4 et 14°C, de préférence entre 4 et 8°C.

24. Procédé selon l'une quelconque des revendications 18 à 23, **caractérisé en ce que** le procédé principal pour la préparation d'un levain-chef à base de froment ou d'un levain de panification légèrement acidifié à base de froment est compris dans des températures inscrites entre 21 et 27°C, de préférence entre 22 et 26°C et de manière particulièrement préférée de l'ordre de 24°C environ.

25. Procédé selon l'une quelconque des revendications précédentes 18 à 24, **caractérisé en ce que**, parallèlement au produit de type mouture de froment, un ou plusieurs autres produits de type mouture de céréale sont utilisés.

26. Procédé selon la revendication 25, **caractérisé en ce que** le ou les produits de type mouture de froment présentent une teneur en cendres d'environ 550 à 1 050 mg par 100 g de produit de type mouture.

27. Procédé selon l'une quelconque des revendications précédentes 18 à 25, **caractérisé en ce qu'**une étape du procédé s'ajoute au procédé principal en vue de la stabilisation du produit du procédé.

28. Procédé selon la revendication 27, **caractérisé en ce que** la stabilisation des produits de consommation courante est réalisée en ajoutant de l'acide acétique ou du vinaigre et/ou du sel et/ou de la farine, notamment en ajoutant du vinaigre en une concentration finale de 5 à 15 % (p/p) et/ou du sel de cuisine (NaCl) en une concentration finale de 2 à 10 % (p/p) et/ou de la farine en une concentration finale de 10 à 50 % rapportées au levain de panification ou au levain-chef à base de froment.

29. Produit de type levain de panification ou levain-chef à base de froment pouvant être obtenu par le procédé selon la revendication 28.

30. Utilisation d'un produit de type levain de panification selon la revendication 30 pour la préparation de pâtisseries, notamment de pâtisseries à base de froment.
